(19) Europäisches Patentamt
European Patent Office
Office européen des brevets

(11) EP 4 212 560 A1

(12) # EUROPEAN PATENT APPLICATION
published in accordance with Art. 153(4) EPC

(43) Date of publication:
**19.07.2023 Bulletin 2023/29**

(21) Application number: **21866702.0**

(22) Date of filing: **06.09.2021**

(51) International Patent Classification (IPC):
***C08F 255/00*** *(2006.01)* ***C08F 283/06*** *(2006.01)*
***A61L 27/16*** *(2006.01)* ***A61L 27/18*** *(2006.01)*
***C08J 7/12*** *(2006.01)* ***C08J 7/16*** *(2006.01)*

(52) Cooperative Patent Classification (CPC):
**A61L 27/16; A61L 27/18; C08F 255/00; C08F 283/06; C08J 7/12; C08J 7/16**

(86) International application number:
**PCT/JP2021/032589**

(87) International publication number:
**WO 2022/054743 (17.03.2022 Gazette 2022/11)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **10.09.2020 JP 2020152312**

(71) Applicant: **Kyocera Corporation**
**Kyoto-shi Kyoto 612-8501 (JP)**

(72) Inventors:
- **YAMANE, Shihori**
  **Kyoto-shi, Kyoto 612-8501 (JP)**
- **NOGUCHI, Aya**
  **Kyoto-shi, Kyoto 612-8501 (JP)**
- **KYOMOTO, Masayuki**
  **Kyoto-shi, Kyoto 612-8501 (JP)**

(74) Representative: **TBK**
**Bavariaring 4-6**
**80336 München (DE)**

(54) **METHOD FOR FORMING GRAFT LAYER, METHOD FOR PRODUCING COMPOSITE, AND TREATMENT LIQUID FOR FORMING GRAFT LAYER**

(57) Provided is a method that efficiently forms a graft chain having a length the same as or greater than heretofore and/or that efficiently forms a graft layer having a thickness the same as or greater than those resulted from a known technique. A method for forming a graft layer according to an aspect of the present disclosure includes: a contact step of bringing a base member containing a polymer A into contact with a treatment liquid in which a compound B and a polymer C are contained in a solvent D; and, during the contact step, a polymerization step of graft-polymerizing the compound B onto the polymer A that constitutes at least a portion of the surface of the base member.

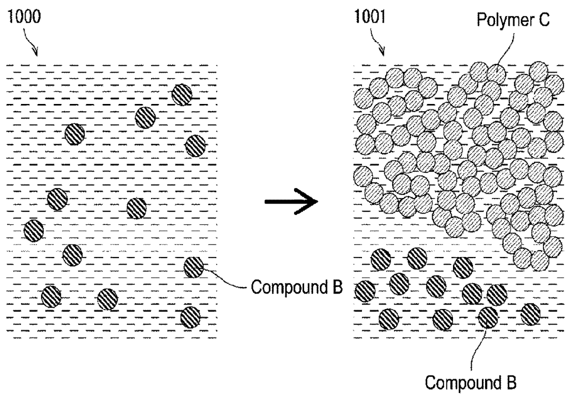


FIG. 1

EP 4 212 560 A1

## Description

TECHNICAL FIELD

The present disclosure relates to a method for forming a graft layer, a method for producing a composite, and a treatment liquid for forming a graft layer.

BACKGROUND OF INVENTION

There is a known technique for forming a polymer film by graft-polymerizing a compound onto the surface of a base member. There is also a known method for forming a polymer film by using an aqueous solution for treatment containing a water-soluble inorganic salt.

SUMMARY

A method for forming a graft layer according to an aspect of the present disclosure includes a contact step of bringing a base member containing a polymer A into contact with a treatment liquid in which a compound B and a polymer C are contained in a solvent D. The contact step includes a polymerization step of graft-polymerizing the compound B onto the polymer A that constitutes at least a portion of the surface of the base member.

A treatment liquid according to an aspect of the present disclosure is a treatment liquid in which a compound B and a polymer C are contained in a solvent D, the treatment liquid being for forming a graft layer in which the compound B is graft-polymerized onto at least a portion of the surface of a base member containing a polymer A.

BRIEF DESCRIPTION OF THE DRAWINGS

FIG. 1 is a schematic view illustrating an excluded volume effect.
FIG. 2 is a schematic view illustrating a gel effect.
FIG. 3 is a schematic view of an artificial hip joint 1 according to an embodiment of the present disclosure.
FIG. 4 is a schematic view of an acetabular cup according to an embodiment of the present disclosure.
FIG. 5 is a graph illustrating a relationship between a concentration of 2-methacryloyloxyethyl phosphorylcholine in a treatment liquid and a static water contact angle in Example 1 and Comparative Example 1.
FIG. 6 is a graph illustrating a relationship between a concentration of 2-methacryloyloxyethyl phosphorylcholine in a treatment liquid and a thickness of the graft layer in Example 1 and Comparative Example 1.

DESCRIPTION OF EMBODIMENTS

An embodiment of the present disclosure will be described in detail below. Unless otherwise specified in the present specification, "A to B" representing a numerical value range means "A or more and B or less".

1. Method for forming graft layer

A method for forming a graft layer according to an embodiment of the present disclosure includes a contact step of bringing a base member containing a polymer A into contact with a treatment liquid in which a compound B and a polymer C are contained in a solvent D. The contact step includes a polymerization step of graft-polymerizing the compound B onto the polymer A that constitutes at least a portion of the surface of the base member.

In the present description, a polymer obtained by polymerizing the compound B is referred to as a "polymer B". In the present description, the term "graft layer" means a layer formed by graft-polymerizing the polymer B onto the base member. In other words, the graft layer is a layer that is formed on the surface of the base member and that contains the polymer B. The graft-polymerized polymer B is also referred to as a "graft chain".

With the above configuration, a graft layer containing the polymer B can be efficiently formed on at least a portion of the surface of the base member. Specifically, the efficiency of graft polymerization of the compound B is improved by an excluded volume effect and a gel effect brought about by the polymer C contained in the solvent D. The excluded volume effect and the gel effect are described below.

FIG. 1 is a schematic view illustrating an excluded volume effect. The polymer C has a volume in the treatment liquid. In general, repulsive force acts between the polymers C, avoiding the polymers C from getting close to each other. Therefore, as illustrated in FIG. 1, the region in which the compound B can be present is smaller in a treatment liquid

1001 with the polymer C added than in a treatment liquid 1000 without the polymer C added. This is called the excluded volume effect. As a result, the apparent concentration of the compound B in the treatment liquid and/or the consumption rate of the compound B increases, and therefore the graft polymerization efficiency of the compound B is improved.

FIG. 2 is a schematic view illustrating a gel effect. As described above, the treatment liquid contains the polymer C, and thus the treatment liquid as a whole has a high viscosity. As a result, the movement of the polymer B having a growing radical is restricted, thereby reducing termination reaction, which is a bimolecular reaction between the polymers B, as shown in FIG. 2. That is, when the polymers B having a growing radical react with each other, further polymerization at the terminal of the polymers B can be terminated, but when the treatment liquid has a high viscosity, the termination of polymerization is reduced. This is called the gel effect. This increases the apparent consumption rate of the compound B.

Because of the two factors described above, a graft chain having a length the same as or greater than heretofore and/or a graft layer having a thickness the same as or greater than heretofore can be efficiently formed on the surface of the base member containing the polymer A while the concentration of the compound B is less than heretofore.

1-1. Treatment liquid

A treatment liquid according to an embodiment of the present disclosure includes the compound B, the polymer C, and the solvent D, the treatment liquid being for forming a graft layer in which the compound B is graft-polymerized on at least a portion of the surface of the base member containing the polymer A. The treatment liquid may contain the polymer C in addition to the compound B at a stage before graft polymerization starts.

By forming a graft layer using the treatment liquid according to an embodiment of the present disclosure, the amount of the compound B used and the amount of the compound B discarded can be reduced as compared with a case using a known technology. In other words, the production efficiency can be improved, and the burden on the environment can be reduced. In addition, for example, the amount of polymerization initiator used can be reduced, the intensity of light irradiated during the photoinitiated graft polymerization can be reduced, and the polymerization temperature during the thermally initiated graft polymerization can be reduced. Therefore, reduction of deterioration of base members due to light irradiation, range expansion of applicable compounds, and the like can be expected.

The polymer B is formed by polymerization of the compound B. Also, the compound B forms the graft layer by graft polymerization. The compound B may be of one type or of multiple types.

The compound B may be electrically neutral. Thereby, the intramolecular interaction of the compound B and/or intermolecular interaction of the compound B can be reduced. In the present description, the term "electrically neutral" means that there are no groups that dissociate into ions in an aqueous solution having a pH value near neutral (pH6 to 8), or that even when there are groups that dissociate into ions, such groups include groups that become a cation and groups that become an anion, and the sum of electric charges is substantially 0. Here, the term "substantially" means that the sum of electric charges is 0, or that even when the sum is not 0, the sum is small enough to not adversely affect the effect of the present disclosure.

The compound B may have a phosphorylcholine group. This allows the graft layer to maintain a high biocompatibility and/or a good lubrication for a long period of time.

The compound B may further have a polymerization-initiating group. For example, the compound B may be a polymerizable monomer having a phosphorylcholine group at one terminal and a polymerization-initiating group capable of graft polymerization with the base member at one of the other terminals.

The compound B may have a polymerizable methacrylic acid unit as the polymerization-initiating group. This makes it possible to easily form the graft layer.

Examples of the compound B having a phosphorylcholine group include 2-methacryloyloxyethyl phosphorylcholine, 2-acryloyloxyethyl phosphorylcholine, 4-methacryloyloxybutyl phosphorylcholine, 6-methacryloyloxyhexyl phosphorylcholine, and ω-methacryloyloxyethylene phosphorylcholine. Hereinafter, 2-methacryloyloxyethyl phosphorylcholine is also referred to as "MPC". A polymer in which MPC is polymerized is also referred to as poly (MPC) or PMPC.

MPC, which has a chemical structure represented by the following structural formula, is a polymerizable monomer having a phosphorylcholine group and a polymerizable methacrylic acid unit.

[Chem. 1]

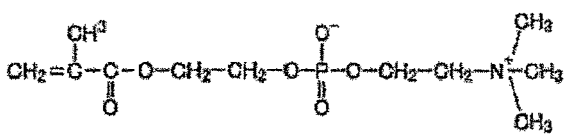

MPC can be easily polymerized by radical polymerization, thus forming a high molecular weight homopolymer (Ishihara

et al., Polymer Journal 22, p355 (1990)). Therefore, forming a graft layer as an aggregate of polymer chains in which MPC is polymerized allows graft-bonding between the MPC polymer chains and the surface of the base member to be performed under relatively mild conditions. Further, forming a high-density graft chain and/or graft layer allows a large number of phosphorylcholine groups to be formed on the surface of the base member.

The graft layer described above can be formed not only as a homopolymer composed of a single polymerizable monomer having a phosphorylcholine group but also as a copolymer composed of a polymerizable monomer having a phosphorylcholine group and, for example, another vinyl compound monomer. This enables a function for improving mechanical strength and the like to be imparted to the graft layer depending on the type of the other vinyl compound monomer used.

Other examples of the compound B include polyethylene glycol dimethacrylate and a monomer having a betaine structure (methacryloyloxyethyl carboxybetaine, methacryloyloxyethyl sulfobetaine, and methacryloyloxyethyl amidobetaine).

A concentration of the compound B in the treatment liquid can be appropriately changed depending on the type of the compound B, and may be, for example, from 0.05 to 0.25 mol/L, from 0.10 to 0.25 mol/L, or from 0.10 to 0.20 mol/L. When the concentration of the compound B is within the above range, the production costs and the environmental impact can be reduced, a graft layer having sufficient density and thickness can be formed, and the wettability of the surface of the graft layer and the wear resistance can be improved.

The polymer C provides the excluded volume effect and the gel effect as described above. The polymer C is not limited as long as it is a polymer that does not interfere with the graft polymerization of the compound B. The polymer C may be an organic polymer or an inorganic polymer. From the viewpoint of solubility in the solvent D, the polymer C may be an organic polymer. The polymer C may be of one type or of multiple types.

The polymer C may be electrically neutral. The meaning of the term "electrically neutral" is as described above. When the polymer C is electrically neutral, the intramolecular interaction of the polymer C and/or intermolecular interaction of the polymer C can be reduced, and the interaction between the polymer C and the compound B and/or the polymer B can also be reduced.

A weight average molecular weight of the polymer C may be 10000 or greater, or from 10000 to 1000000, or from 100000 to 1000000. With this configuration, the excluded volume effect brought about by the polymer C in the treatment liquid is improved, and the efficiency of the graft polymerization of the compound B is improved. The weight average molecular weight can be measured, for example, using gel permeation chromatography.

The polymer C may have a phosphorylcholine group. The monomer constituting the polymer C may be the same compound as the compound B. The polymer C may be, for example, poly(2-methacryloyloxyethyl phosphorylcholine).

Meanwhile, the polymer B and the polymer C may be different compounds that do not react with each other. As a result, the compound B is used only for graft polymerization with respect to the base member, and thus the efficiency of graft polymerization of the compound B can be improved.

In addition to the polymer having a phosphorylcholine group, examples of the polymer C include polymethacrylic acid polyethylene glycol, various polymers having a betaine group, starch, sucrose, and hyaluronic acid.

A concentration of the polymer C in the treatment liquid can be appropriately changed depending on the type of the polymer C, and may be, for example, 1 μmol/L or greater, or from 1 to 1000 μmol/L. When the concentration of the polymer C is within the above range, the excluded volume effect brought about by the polymer C in the treatment liquid can be improved, and the efficiency of the graft polymerization of the compound B can be improved. Further, even when the polymer B is used as the polymer C, the amount of the compound B to be discarded can be reduced compared to when the polymer B is not used.

Furthermore, a dissolved oxygen concentration in the treatment liquid before the start of graft polymerization may be 6.0 mg/L or less, or may be 0.2 mg/L or less. When the dissolved oxygen concentration is within the above range, inhibition of polymerization of the compound B due to dissolved oxygen can be reduced.

The solvent D is not limited, and may be a hydrophilic solvent or a hydrophobic solvent. From the viewpoint of the burden on the environment, the solvent may be a hydrophilic solvent. Examples of the hydrophilic solvent include water, salt solution, sugar solution, and a water/ethanol mixed solution. Examples of the hydrophobic solvent include alcohol, acetone, and hexane. The solvent D may contain at least water.

The solvent D may be a good solvent for the polymer B and/or the polymer C, the polymer B being resulted from polymerization of the compound B. The solvent D may be a good solvent for both the polymer B and the polymer C.

In the present description, the term "good solvent" refers to a solvent in which the solubility of a target compound is relatively greater than the solubility of the target compound in a poor solvent described below. With this configuration, a large amount of the polymer B and/or the polymer C can be dissolved in the solvent, and thus the efficiency of graft polymerization can be improved.

Further, the solvent D may be a good solvent for the compound B. When the solvent D is a good solvent for the compound B, the mobility of the compound B in the solvent D can be improved, and thus the efficiency of graft

polymerization of the compound B can be improved.

For the purpose of recovering polymer produced by polymerization from the solvent, a poor solvent may be used as the solvent. However, in the method for forming a graft layer according to an embodiment of the present disclosure, a good solvent for the polymer B can be used as described above.

The treatment liquid may further contain an inorganic salt that is soluble in the solvent D. This can improve the efficiency of graft polymerization of the compound B.

When the solvent D is a hydrophilic solvent, a water-soluble inorganic salt may be used as the inorganic salt. Examples of the water-soluble inorganic salt include an alkali metal salt and an alkaline earth metal salt. Examples of the alkali metal salt include a sodium salt, a potassium salt, a lithium salt, and a cesium salt. Examples of the alkaline earth metal salt include a magnesium salt, a calcium salt, a strontium salt, a barium salt, and a radium salt. Further, examples of the inorganic salt, if classified according to the type of counter anion, include halides (for example, chloride, fluoride, bromide, and iodide), phosphates, carbonates, nitrates, and hydroxides. The water-soluble inorganic salt is one or more selected from the group consisting of, for example, sodium chloride, potassium chloride, calcium chloride, and magnesium chloride.

A concentration of the inorganic salt in the treatment liquid may be, for example, from 0.01 to 5.0 mol/L, from 1.0 to 5.0 mol/L, or from 1.0 to 3.0 mol/L. The above concentration allows a graft layer having sufficient graft density to be efficiently formed.

1-2. Base member

The base member is a target onto which the graft layer is formed. The base member may include the polymer A on at least a portion of its surface. The base member may include functional compounds, such as antioxidants and crosslinking agents, and/or reinforcing materials, such as carbon fibers.

Examples of the polymer A include a polyolefin and an aromatic polyether ketone. The polymer A may be of one type or of multiple types. Examples of the polyolefin include polyethylene. For example, from the viewpoint of excellent mechanical characteristic such as wear resistance, impact resistance, and deformation resistance, examples of the polyethylene include an ultra-high molecular weight polyethylene (UHMWPE). From the viewpoint of excellent mechanical characteristic such as impact resistance and deformation resistance, examples of the aromatic polyether ketone include polyether ether ketone (PEEK).

The polymer A may contain a free radical. In the present description, the term "free radical" refers to a molecule that has an unpaired electron and that is paramagnetic. A content of the free radical can be measured by electron spin resonance. An amount of the free radical may be $1.0 \times 10^{14}$ spins/g or greater, from $1.0 \times 10^{14}$ to $1.0 \times 10^{20}$ spins/g, or from $1.0 \times 10^{15}$ to $1.0 \times 10^{20}$ spins/g.

The higher the molecular weight of the polymer constituting the base member is, the higher the wear resistance tends to be. When the base member includes a polyolefin, the molecular weight of the polymer constituting the base member may be 1000000 or greater, from 1000000 to 7000000, from 3000000 to 7000000, or from 3000000 to 4000000. When the base member includes an aromatic polyether ketone, the molecular weight of the polymer constituting the base member may be 50000 or greater, from 80000 to 500000, or from 80000 to 200000. In the present description, the molecular weight of the polymer constituting the base member means the molecular weight determined by Equation (1) below by measuring the viscosity of a decahydronaphthalene (decalin) solution containing the polymer at 135°C.

$$\text{Molecular weight} = 5.37 \text{ x } 10^4 \text{ x (intrinsic viscosity)}^{1.49} \text{ ...(1)}$$

From the viewpoint of mechanical characteristics such as impact resistance and deformation resistance, a density of the polymer constituting the base member may be from 0.927 to 0.944 g/cm$^3$ when the base member includes a polyolefin. Also, when the base member includes an aromatic polyether ketone, the density may be from 1.20 to 1.55 g/cm$^3$.

1-3. Contact step

The contact step is a step of bringing the base member containing the polymer A into contact with the treatment liquid in which the compound B and the polymer C are contained in the solvent D. In the contact step, at least a portion of the base member may be brought into contact with the treatment liquid. For example, a portion of the surface of the base member with the polymer A present may be brought into contact with the treatment liquid, or the entire base member may be brought into contact with the treatment liquid.

A method of bringing the base member into contact with the treatment liquid is not limited, and any method can be used. From the viewpoint of efficiently forming the graft layer, a method of immersing the base member in the treatment

liquid may be used. A contact time between the base member and the treatment liquid is not limited, but may be 5 minutes or longer from the viewpoint of performing the polymerization step described later.

1-4. Polymerization step

The polymerization step is a step of, during the contact step, graft-polymerizing the compound B onto the polymer A that constitutes at least a portion of the surface of the base member. The polymerization step may be carried out simultaneously with the contact step. A method of graft polymerization is not limited, and may be, for example, photoinitiated graft polymerization or thermally initiated graft polymerization.

When the method of graft polymerization is photoinitiated graft polymerization, the polymer B resulted from polymerization of the compound B can be stably immobilized on the surface of the base member. Further, the photoinitiated graft polymerization causes the polymer B to be formed at a high density on the surface of the base member, thus increasing the density of the graft layer.

The photoinitiated graft polymerization may be initiated by visible light or by ultraviolet light. When the surface of the base member is irradiated with ultraviolet light, the compound B in the vicinity of the surface polymerizes to produce the polymer B. The produced polymer B is covalently bonded to the surface of the base member. The polymer B is graft-bonded to the surface at a high density, thus forming a graft layer covering the entire surface of the base member. At this time, the base member may be heated. By heating the base member and the treatment liquid in contact with the base member, the photoinitiated graft polymerization can be controlled.

The surface of the base member may contain a photopolymerization initiator. For example, before the base member is brought into contact with the treatment liquid, a photopolymerization initiator may be applied to the surface of the base member. In this case, a photopolymerization initiator radical generated by the ultraviolet irradiation forms a polymerization initiation point on the surface of the base member. The compound B reacts with the polymerization initiation point to initiate graft polymerization and grows into the polymer B.

The wavelength of the ultraviolet rays to be irradiated is, for example, 300 to 400 nm. Examples of ultraviolet irradiation sources that can be used include high-pressure mercury lamps (UVL-400HA, available from Riko Kagaku Sangyo Co., Ltd.) and LEDs (MeV365-P601JMM, available from YEV Co., Ltd.). The ultraviolet irradiation time may be 11 to 90 minutes or may be 23 to 90 minutes.

A heating temperature and heating time of the thermally initiated graft polymerization are not limited, but the heating temperature may be equal to or lower than the melting point of the polymer A and/or the polymer B and/or the polymer C, and may be equal to or lower than the boiling point of the solvent D. The heating temperature may be, for example, from 25 to 150°C, and the heating time may be, for example, from 10 to 180 minutes.

The graft polymerization may also be initiated by irradiation with gamma rays. A time of irradiation with gamma rays is not limited, and may be, for example, from 5 to 120 minutes.

After completion of the graft polymerization, the treatment liquid may be removed by washing. In addition, sterilization treatment using gamma-ray irradiation, ethylene oxide gas, or the like may be further performed.

2. Method for producing composite

A method for producing a composite according to an embodiment of the present disclosure is a method for producing a composite including a base member and a graft layer covering at least a portion of a surface of the base member. The method for producing the composite includes forming a graft layer in which the compound B is graft-polymerized onto at least a portion of the surface of the base member containing the polymer A using the method for forming a graft layer described above. The matters already described in "1. Method for forming graft layer" will not be described below.

2-1. Base member forming step

The base member in the production method may be a commercially available product, or the production method may include a base member forming step before the step of forming a graft layer. The base member can be obtained by, for example, placing the polymer A that is powdery, granular, or pellet-like into a metal mold, followed by compression molding, extrusion molding, or injection molding. Examples of the polymer A include the UHMWPE and the PEEK described above. The UHMWPE and the PEEK, which are thermoplastic resins, have less flowability than the melting temperature. Therefore, the UHMWPE or the PEEK in a solid state may be charged into a metal mold and molded under high heat and pressure conditions. An antioxidant, a crosslinking agent, or a reinforcing material such as carbon fiber may be added to the metal mold together with the polymer A.

2-2. Crosslinking step

The method for producing a composite according to an embodiment of the present disclosure may include a crosslinking step of generating a crosslinked structure in the molecule of the polymer A before the step of forming a graft layer, for example, between the base member forming step and the step of forming a graft layer. This obtains a base member having further improved mechanical characteristics, such as wear resistance.

The crosslinking step may include irradiating the base member with a high energy ray. This step is also referred to as a high energy ray irradiation step. The base member is irradiated with the high energy ray to generate a free radical. This causes the polymer A to be bonded between molecular chains, producing a polymer A having a crosslinked structure. Generating the crosslinked structure between the molecular chains improves the mechanical characteristics, such as wear resistance and impact resistance.

The crosslinking reaction is made possible by adding a crosslinking agent, but completely removing unreacted crosslinking agent tends to be difficult. Therefore, the crosslinking reaction by high energy ray irradiation may be used in consideration of the influence of the unreacted crosslinking agent on the living body.

Examples of the high energy ray include X-rays, gamma rays, and electron beams. An irradiation dose of the high energy ray may be, for example, from 25 to 200 kGy, or may be from 50 to 150 kGy. Examples of the high energy ray source that can be used include a radiation device using Co (cobalt) 60 as a radiation source as a gamma ray source, an accelerator that emits an electron beam, and a device that emits an X-ray.

The crosslinking step may further include a thermal treatment step after the high energy ray irradiation step. In the thermal treatment step, the free radical generated by the high energy ray irradiation step is more efficiently consumed in the crosslinking reaction to promote intramolecular crosslinking. The temperature range of the thermal treatment may be 110 to 130°C. The thermal treatment time may be 2 to 12 hours.

3. Use of composite

The composite produced by the production method can be used as, for example, a member of a medical device, a member of an industrial device, or the like. Examples of the member of a medical device include a member of an artificial joint, an artificial blood vessel, an artificial heart, and various stents.

The artificial joint to which the member of an artificial joint is applied is not limited. Examples of the artificial joint include an artificial hip joint, an artificial knee joint, an artificial ankle joint, an artificial shoulder joint, an artificial elbow joint, an artificial finger joint, and an artificial intervertebral disc. For example, the artificial hip joint may include a femoral head and an acetabulum. The member of an artificial joint according to an embodiment of the present disclosure can be applied to the femoral head or the acetabulum, or both. For example, when the member of an artificial joint is used in one of the femoral head and the acetabulum, the other one may use a member including a metal such as stainless steel or a cobalt chromium alloy, a ceramic such as alumina or zirconia, or a polymer such as the UHMWPE or the PEEK. Also, for example, the femoral head and acetabulum may be formed of different materials. For example, the femoral head may be formed of a polymer, ceramic, or metal material, and the base member of the acetabulum may be formed of, for example, a polymeric material.

Hereinafter, an example in which the composite according to an embodiment of the present disclosure is used as a member of an artificial joint and applied to an acetabular cup of an artificial hip joint will be described. FIG. 3 is a schematic view of an artificial hip joint 1 according to an embodiment of the present disclosure. FIG. 4 is a schematic view of an acetabular cup 10 according to an embodiment of the present disclosure. The artificial hip joint 1 is composed of the acetabular cup 10 to be fixed to an acetabulum 94 of a hip bone 93 and a femoral stem 20 to be fixed to a proximal end of a femur 91. The acetabular cup 10 includes a cup base member 12, which has a substantially hemispherical acetabular fixing surface 14 and a substantially hemispherically recessed sliding surface 16, and a graft layer 30, which covers the sliding surface 16. A femoral head 22 of the femoral stem 20 fits into and slides within a recess 161 in which the graft layer 30 of the acetabular cup 10 is formed; in this way, the artificial hip joint 1 functions as a hip joint. The acetabular fixing surface 14 is an outer surface disposed closer to the acetabulum 94. The sliding surface 16 is also an inner surface or a contact surface in contact with the femoral head 22.

As illustrated in FIGs. 3 and 4, in the acetabular cup 10, the sliding surface 16 of the cup base member 12 is covered with the graft layer 30. The graft layer 30 is obtained by graft-polymerizing the polymer B, resulted from polymerization of the compound B, onto the sliding surface 16. The graft layer 30 may be disposed only on the acetabular cup 10 and may be disposed on both the acetabular cup 10 and the femoral head 22.

The graft layer 30 has a structure similar to that of a biological film, has a high affinity with lubricating liquid in the joint, and can retain the lubricating liquid inside the film. Furthermore, the graft layer 30 includes a phosphate group at a high density. Thus, the acetabular cup 10 exhibits superior wear resistance.

EXAMPLE

Hereinafter, an aspect of the present disclosure will be described in more detail based on examples and comparative examples, but the aspects according to the present disclosure are not limited to these examples.

Example 1

2-methacryloyloxyethyl phosphorylcholine (MPC) monomer was used as the compound B, poly(MPC) (PMPC) was used as the polymer C, and pure water was used as the solvent D. The polymer C had a weight average molecular weight of from 20 to 1000000. PMPC, NaCl, and MPC were dissolved in pure water to prepare a treatment liquid. In the treatment liquid, the PMPC concentration was 10 μmol/L, the NaCl concentration was 2.5 mol/L, and the MPC concentration was 0.05 mol/L. An ultra-high molecular weight polyethylene having a molecular weight of from 3000000 to 4000000 and a density of 0.93 g/cm$^3$ was used as the polymer A. A square member (cross section: 10 mm × 3 mm, length: 50 mm) made of the polymer A was used as a base member. The square member was immersed in the prepared treatment liquid and then irradiated with ultraviolet light for 90 minutes. After completion of the ultraviolet light irradiation, the square member was lifted from the treatment liquid and sufficiently washed with pure water and ethanol, resulting in a test piece having a graft layer of PMPC formed on the surface of the base member.

In addition, test pieces were prepared by the same method as described above using treatment liquids in which the MPC concentration was changed to 0.08 mol/L, 0.1 mol/L, 0.15 mol/L, 0.2 mol/L, 0.25 mol/L, and 0.5 mol/L.

Comparative Example 1

Test pieces were prepared in the same manner as in Example 1 except that the polymer C was not added to the treatment liquids. In addition, a test piece using a treatment liquid to which neither the compound B nor the polymer C was added was also prepared in Comparative Example 1.

Measurement of static water contact angle

The hydrophilicity of each of the test pieces was evaluated by measuring the contact angle (static water contact angle) when pure water was dropped on the surface of each test piece with the graft layer formed. The static water contact angles were evaluated by the droplet method using a surface contact angle measuring device (DM300, available from Kyowa Interface Science Co., Ltd.). More specifically, in accordance with the ISO15989 standard, pure water with a droplet volume of 1 μL was dropped onto the surfaces of the test pieces, and the contact angles were measured after 60 seconds.

Measurement of thickness of graft layer

Each of the test pieces was embedded in epoxy resin and then stained with ruthenium tetrachloride. Thereafter, an ultra-thin piece was cut from the test piece using an ultramicrotome. An electron microscopic image of the cross section of the ultra-thin piece was obtained using a transmission electron microscope (TEM) with the accelerating voltage set to 100 kV. For each of the electron microscopic images obtained, the film thickness on the cross section was measured at 10 points, and the average value thereof was calculated as the thickness of the graft layer.

[Evaluation Result]

FIG. 5 is a graph illustrating a relationship between the concentration of MPC in the treatment liquid and the static water contact angle in Example 1 and Comparative Example 1. In FIG. 5, the black circles (polymer C (+)) indicate the results of the test pieces of Example 1 prepared with the polymer C added, and the white circles (polymer C (-)) indicate the results of the test pieces of Comparative Example 1 prepared without the polymer C added. FIG. 5 indicates that, in Example 1, a graft layer having high hydrophilicity could be formed even when the concentration of the compound B in the treatment liquid was low. In particular, the contact angles of the test pieces prepared using treatment liquids having an MPC concentration of from 0.08 to 0.25 mol/L had a low value of 45° or less. In addition, the contact angles of the test pieces of Examples prepared using treatment liquids having an MPC concentration of from 0.1 to 0.2 mol/L were particularly low.

FIG. 6 is a graph illustrating a relationship between the concentration of MPC in the treatment liquid and the thickness of the graft layer in Example 1 and Comparative Example 1. The black circles and white circles in FIG. 6 have the same meanings as those in FIG. 5. FIG. 6 indicates that, in Example 1, even when the concentration of MPC in the treatment liquid was low, a graft layer having a thickness equal to or greater than that of a graft layer formed using a

known technique could be formed. In particular, when the MPC concentration in the treatment liquid was from 0.08 to 0.25 mol/L, the thicknesses of the graft layers were from 50 to 250 nm and were greater than those of the test pieces of Comparative Example 1 prepared without the polymer C added.

In the present disclosure, the invention has been described above based on the various drawings and examples. However, the invention according to the present disclosure is not limited to each embodiment described above. That is, the embodiments of the invention according to the present disclosure can be modified in various ways within the scope illustrated in the present disclosure, and embodiments obtained by appropriately combining the technical means disclosed in different embodiments are also included in the technical scope of the invention according to the present disclosure. In other words, note that a person skilled in the art can easily make various variations or modifications based on the present disclosure. Note that these variations or modifications are included within the scope of the present disclosure.

## INDUSTRIAL APPLICABILITY

The invention according to the present disclosure can be used as a method for forming a graft layer.

## REFERENCE SIGNS

1 Artificial hip joint
10 Acetabular cup (member of an artificial joint)
12 Cup base member (base member)
30 Graft layer

## Claims

**1.** A method for forming a graft layer comprising:

a contact step of bringing a base member containing a polymer A into contact with a treatment liquid in which a compound B and a polymer C are contained in a solvent D; and
during the contact step, a polymerization step of graft-polymerizing the compound B onto the polymer A that constitutes at least a portion of the surface of the base member.

**2.** The method for forming a graft layer according to claim 1, wherein the polymer C has a weight average molecular weight of 10000 or greater.

**3.** The method for forming a graft layer according to claim 1 or 2, wherein the solvent D is a good solvent for the polymer B and/or the polymer C, the polymer B being resulted from polymerization of the compound B.

**4.** The method for forming a graft layer according to any one of claims 1 to 3, wherein the treatment liquid further comprises an inorganic salt that is soluble in the solvent D.

**5.** The method for forming a graft layer according to any one of claims 1 to 4, wherein the compound B comprises a phosphorylcholine group.

**6.** The method for forming a graft layer according to any one of claims 1 to 5, wherein the compound B comprises a polymerizable methacrylic acid unit.

**7.** The method for forming a graft layer according to any one of claims 1 to 6, wherein the polymer A is a polyolefin or an aromatic polyether ketone.

**8.** A method for producing a composite that comprises a base member and a graft layer covering at least a portion of a surface of the base member, the method comprising forming a graft layer in which a compound B is graft-polymerized onto at least a portion of the surface of a base member comprising a polymer A using the method for forming a graft layer described any one of claims 1 to 7.

**9.** The method for producing a composite according to claim 8, wherein the composite is a member of a medical device.

**10.** The method for producing a composite according to claim 9, wherein the member of a medical device is a member of an artificial joint.

**11.** A treatment liquid in which a compound B and a polymer C are contained in a solvent D,
the treatment liquid being for forming a graft layer in which the compound B is graft-polymerized onto at least a portion of the surface of a base member containing a polymer A.

**12.** The treatment liquid according to claim 11, wherein the polymer C has a weight average molecular weight of 10000 or greater.

**13.** The treatment liquid according to claim 11 or 12, wherein the solvent D is a good solvent for the polymer B and/or the polymer C, the polymer B being resulted from polymerization of the compound B.

**14.** The treatment liquid according to any one of claims 11 to 13, further comprising an inorganic salt that is soluble in the solvent D.

**15.** The treatment liquid according to any one of claims 11 to 14, wherein the compound B comprises a phosphorylcholine group.

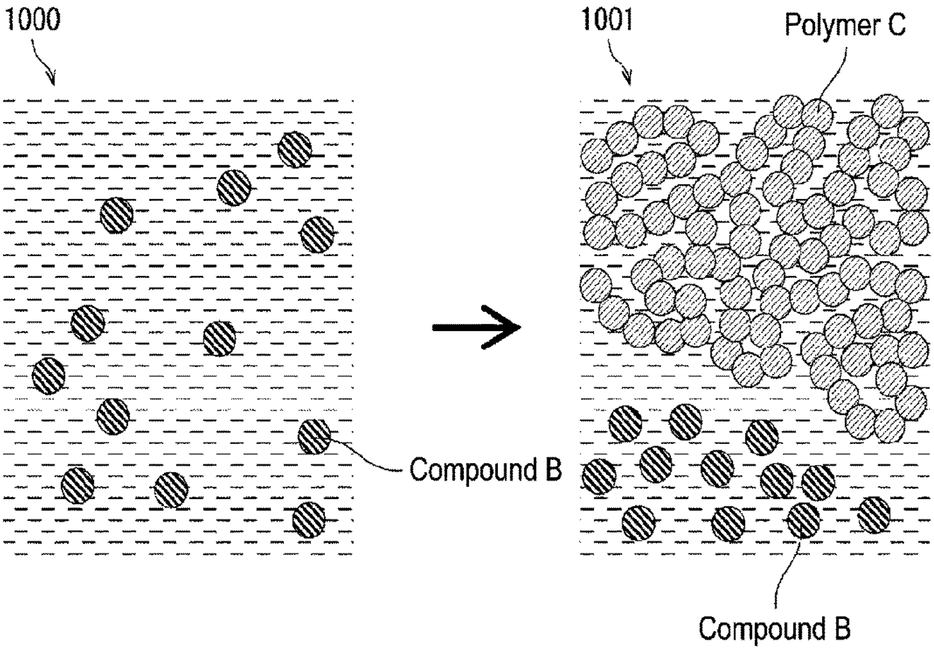


FIG. 1

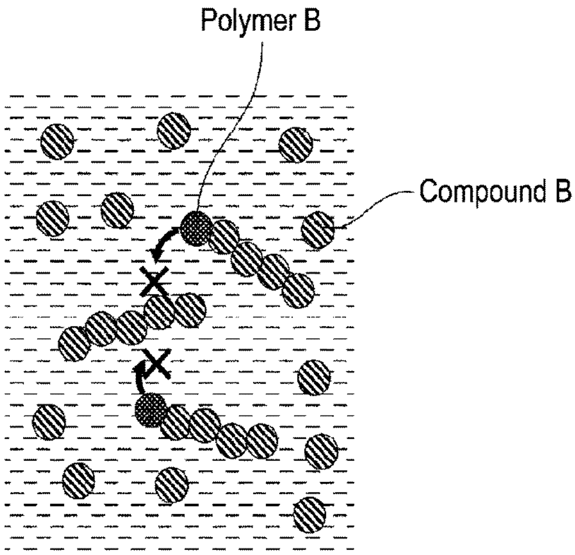


FIG. 2

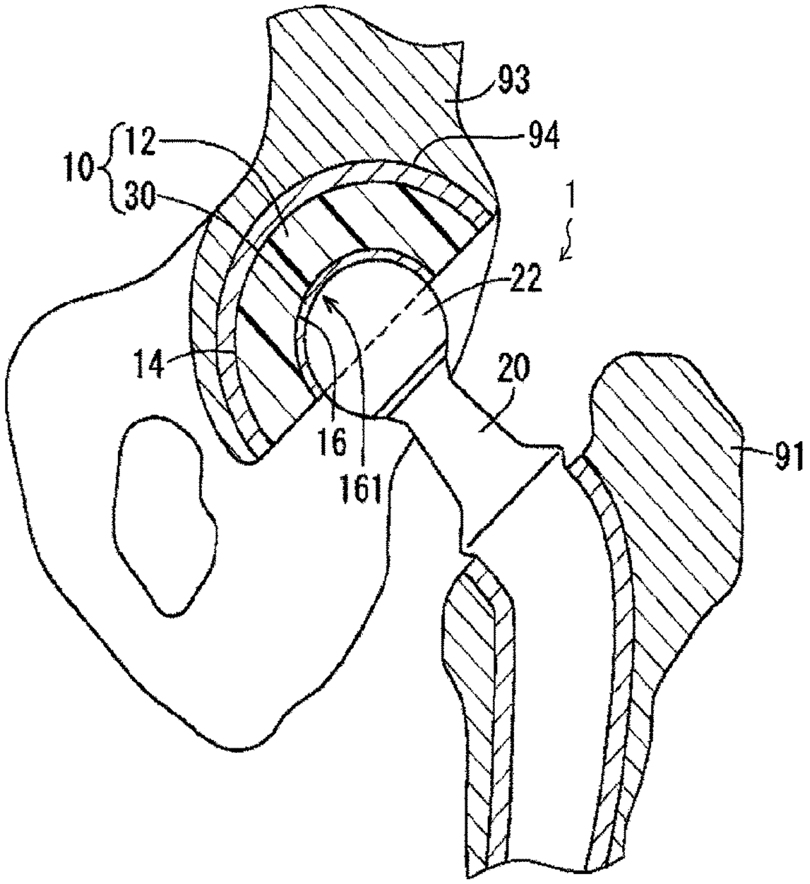


FIG. 3

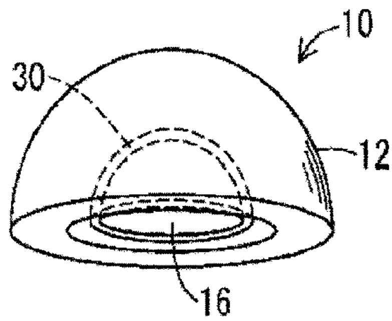


FIG. 4

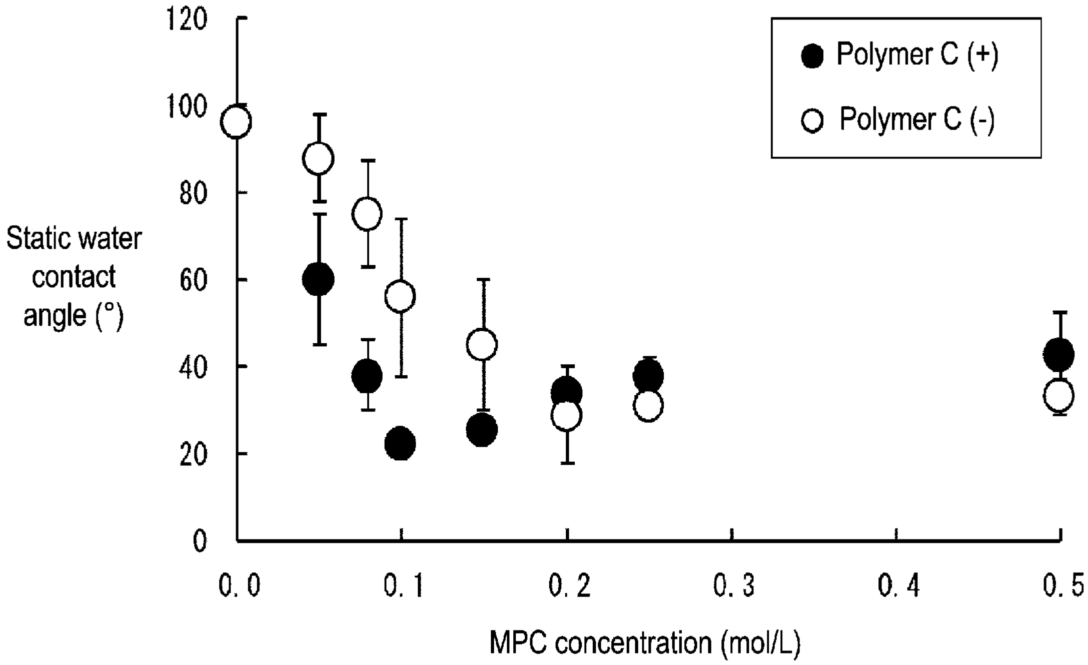
120
100
80
60
40
20
0
Static water contact angle (°)
Polymer C (+)
Polymer C (-)
0. 0
0. 1
0. 2
0. 3
0. 4
0. 5
MPC concentration (mol/L)

FIG. 5

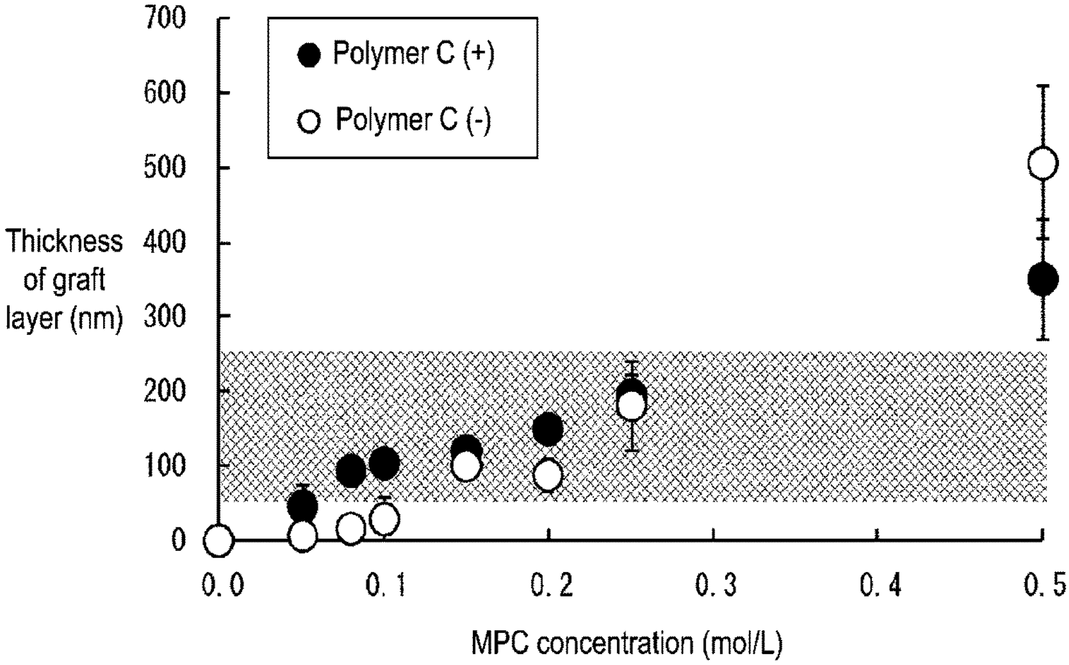
700
600
500
400
300
200
100
0
Thickness of graft layer (nm)
Polymer C (+)
Polymer C (-)
0. 0
0. 1
0. 2
0. 3
0. 4
0. 5
MPC concentration (mol/L)

FIG. 6

## INTERNATIONAL SEARCH REPORT

International application No. **PCT/JP2021/032589**

**A. CLASSIFICATION OF SUBJECT MATTER**

***C08F 255/00***(2006.01)i; ***C08F 283/06***(2006.01)i; ***A61L 27/16***(2006.01)i; ***A61L 27/18***(2006.01)i; ***C08J 7/12***(2006.01)i; ***C08J 7/16***(2006.01)i

FI: C08J7/16; C08F255/00; C08F283/06; A61L27/18; C08J7/12 C; A61L27/16

According to International Patent Classification (IPC) or to both national classification and IPC

**B. FIELDS SEARCHED**

Minimum documentation searched (classification system followed by classification symbols)

C08J7/00-7/18; C08F251/00-283/00;283/02-289/00;291/00-297/08; C08F2/00-2/60; A61L27/16; A61L27/18

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2020-043891 A (KYOCERA CORP) 26 March 2020 (2020-03-26) claims 1, 7, 10-12, paragraphs [0020], [0030], [0047], example 3 | 1-15 |
| X<br>A | JP 11-080394 A (HUELS AKTIENGESELLSCHAFT) 26 March 1999 (1999-03-26) claims 1, 20, paragraphs [0028], [0039], [0042], [0044], [0053]-[0054] | 1-3, 5-13, 15<br>4, 14 |
| A | JP 2017-213218 A (KYOCERA CORP) 07 December 2017 (2017-12-07) claims 1, 4 | 1-15 |
| A | JP 6-087968 A (DOW CORNING CORPORATION) 29 March 1994 (1994-03-29) claims 1-2 | 1-15 |
| A | JP 2013-162796 A (DAINIPPON PRINTING CO LTD) 22 August 2013 (2013-08-22) claims 1, 7, paragraphs [0023]-[0024] | 1-15 |

☐ Further documents are listed in the continuation of Box C.

☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **21 October 2021** | **02 November 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2021/032589**

| Patent document cited in search report | Publication date (day/month/year) | Patent family member(s) | Publication date (day/month/year) |
|---|---|---|---|
| JP 2020-043891 A | 26 March 2020 | WO 2020/054867 A1 | |
| JP 11-080394 A | 26 March 1999 | EP 893164 A2<br>claims 1, 20, paragraphs [0011], [0022], [0024], [0026], [0029]-[0030]<br>DE 19809054 A<br>NO 983012 D<br>CA 2241483 A<br>NO 983012 D0 | |
| JP 2017-213218 A | 07 December 2017 | WO 2017/209222 A1 | |
| JP 6-087968 A | 29 March 1994 | US 5429839 A<br>claim 1<br>US 5527618 A<br>EP 561507 A1<br>CA 2090177 A1 | |
| JP 2013-162796 A | 22 August 2013 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **ISHIHARA et al.** *Polymer Journal,* 1990, vol. 22, 355